Europäisches Patentamt

European Patent Office

Office européen des brevets

(19)

(11) Veröffentlichungsnummer: **0 126 389 B1**

(12) **EUROPÄISCHE PATENTSCHRIFT**

(45) Veröffentlichungstag der Patentschrift:
01.03.89

(51) Int. Cl.⁴: **C 12 M 3/00**, C 12 N 15/00, C 12 N 13/00

(21) Anmeldenummer: **84105313.5**

(22) Anmeldetag: **10.05.84**

(54) **Kammer zur Behandlung von Zellen im elektrischen Feld.**

(30) Priorität: **13.05.83 DE 3317415**

(43) Veröffentlichungstag der Anmeldung:
**28.11.84 Patentblatt 84/48**

(45) Bekanntmachung des Hinweises auf die Patenterteilung:
**01.03.89 Patentblatt 89/9**

(84) Benannte Vertragsstaaten:
**AT BE CH FR GB IT LI LU NL SE**

(56) Entgegenhaltungen:
**DE-B- 2 052 548**

**TRENDS IN BIOTECHNOLOGY, Band 1, Nr. 5, 1983, Seiten 149-155, Elsevier Science Publishers B.V., Amsterdam, NL; U. ZIMMERMAN: "Electrofusion of cells: principles and industrial potential"**
**BIOCHIMICA ET BIOPHYSICA ACTA, Band 694, 1982, Seiten 227-277, Elsevier Biomedical Press, NL; U. ZIMMERMAN: "Electric field-mediated fusion and related electrical phenomena"**
**JOURNAL OF MEMBRANE BIOLOGY, Band 67, 1982, Seiten 165-182, Springer-Verlag New York Inc., New York, US; U. ZIMMERMAN et al.: "Electric field-induced cell-to-cell fusion"**

(73) Patentinhaber: **Kernforschungsanlage Jülich Gesellschaft mit beschränkter Haftung, Postfach 1913, D-5170 Jülich 1 (DE)**

(72) Erfinder: **Matschke, Christian, Wirthstrasse 63, D-5110 Alsdorf (DE)**
Erfinder: **Arnold, William Michael, Adalbertstrasse 116-118, D-5100 Aachen (DE)**
Erfinder: **Büchner, Karl-Heinz, Schützenstrasse 19, D-5170 Jülich (DE)**
Erfinder: **Zimmermann, Ulrich, Prof., im Geyberg 21, D-5165 Hürtgenwald-Gey (DE)**

## Beschreibung

Die Erfindung bezieht sich auf eine Kammer für die Behandlung von Zellen im elektrischen Feld, bei der ein aus elektrisch nichtleitenden Wänden gebildeter Raum zur Aufnahme der die Zellen enthaltenden Suspension vorgesehen ist, in den wenigstens zwei Elektroden derart hineinragen, daß ein zwischen den Elektroden liegender, durch diese begrenzter Bereich gebildet wird, in welchem die Zellen einem zwischen den Elektroden ausgebildeten elektrischen Feld ausgesetzt sind.

Aus der DE-PS-2 405 119 ist ein Verfahren zur Behandlung von Zellen im elektrischen Feld bekannt, bei dem die Membran von Zellen durch Anwendung eines elektrischen Feldes, dessen Stärke $10^3$ bis $10^5$ V/cm beträgt, durchbrochen wird. Die dabei bewirkte Permeabilitätserhöhung der Zellmembran macht es möglich, Stoffe durch die Membran auszutauschen, ohne daß die Zellen in ihrer Lebensfähigkeit beeinträchtigt sind. Denn die Erhöhung der Permeabilität ist nach dem Austausch der Stoffe in einem einfachen Verfahrensschritt reversibel. Auf diese Weise ist es beispielsweise auch möglich, Gene oder Enzyme in die Zellen einzulagern.

Das aus der vorgenannten Patentschrift bekannte Verfahren der Erhöhung der Permeabilität der Zellmembran ist auch zur Fusion von Zellen einsetzbar.

Zwei Zellen in einer Suspension sollten, wenn sie sich berühren und ein enger Kontakt zwischen den Membranen beider Zellen entsteht, miteinander verschmelzen, da die Bausteine in der Membran beweglich sind. Eine derartige spontane Verschmelzung (Fusion) von Zellen wird unter natürlichen Bedingungen jedoch nicht oder nur äußerst selten beobachtet. Eine bekannte Ausnahme stellt die Befruchtung einer Eizelle durch eine Samenzelle bei der sexuellen Fortpflanzung dar. Die spontane Fusion wird durch die negative Ladung der Phospholipide und anderer Membrankomponenten behindert. Sie führt zur Abstoßung der Zellen, wenn sie sich auf einen geringen Abstand genähert haben. Zellverschmelzung erfordert aber, daß die beiden Membranen sich bis auf einen Abstand von weniger als $10^{-7}$ cm nähern können.

Die mit technischen Mitteln, also auf künstlichem Wege durchgeführte Fusion von Zellen ist in einem weiten Anwendungsbereich einsetzbar. So ist es für die biologisch-medizinische Forschung von großem Interesse, eine große Zahl von Zellen miteinander zu verschmelzen. Bei geeigneter Größe der durch Fusion mehrerer oder gegebenenfalls vieler Zellen – beispielsweise 1000 bis 10 000 Blutkörperchen – entstandenen großen Zellen lassen sich dann Mikroelektroden, Mikrodruckmeßsonden und andere Sensoren ohne irreversible Zerstörung der Membran in die große Zelle einführen. Die Technik, über die Sensoren direkt eine Reihe von Zellen und Membranfunktionen zu erfassen, ist dabei für die klinische Diagnostik, z. B. bei der Früherkennung von Erkrankungen sowie generell für die Grundlagenforschung von Bedeutung.

Die Technik der Fusion von Zellen kann außerdem eingesetzt werden für die Bildung von Hybridzellen durch Verschmelzung von zwei Zellen unterschiedlicher Herkunft, die evolutionsmäßig nicht zu weit voneinander entfernt stehen sollen. Dabei können Zellhybride aus Pflanzenzellen, aus denen wieder ganze Pflanzen gezüchtet werden können oder Zellhybride aus tierischen Zellen, über die monoklonale Antikörper, z. B. gegen Tumore und Leukämie, gewonnen werden können, gebildet werden. Als Beispiel sei genannt die Verschmelzung einer Lymphozytenzelle mit einer Myelomzelle, die besonders aus medizinischer und pharmazeutischer Sicht von großem Interesse ist. Bestimmte Lymphozyten bilden gegen Fremdstoffe im Organismus Antikörper, z. B. gegen ein Fremdeiweiß, das in die Blutbahn injiziert worden ist. Isoliert man die Lymphozyten und fusioniert sie mit einer Tumorzelle, wie der Myelomzelle, so besteht die Chance, daß sich eine sogenannte Hybridomzelle bildet, die die Eigenschaft beider Elternzellen besitzt. Diese Zelle produziert Antikörper, und zwar spezifisch nur gegen den betreffenden Fremdstoff (sogenannte monoklonale Antikörper). Sie ist unsterblich und läßt sich im Gegensatz zu einer normalen ausdifferenzierten Zelle, wie dem Lymphozyten, permanent in Nährmedien vermehren.

Ein Verfahren zur Fusion von Zellen der eingangs bezeichneten Art ist aus Biochimica et Biophysica Acta, 694 (1982), 227–277 (Electric Field-Mediated Fusion And Related Electrical Phenomena, U. Zimmermann) bekannt. Bei diesem bekannten Verfahren – dessen Ablauf unter dem Mikroskop beobachtet werden kann – wird der Membrankontakt zwischen wenigstens zwei Zellen durch Anlegen eines alternierenden, schwach homogenen Feldes erzeugt. Durch das elektrische Feld werden, bedingt durch Polarisationsprozesse in der Zelle, Dipole erzeugt, die sich gegenseitig anziehen, wenn sich die Zellen während ihrer Wanderung im elektrischen Feld einander nähern (sogenannte Dielektrophorese). Nach der Bildung der Zellenreihe werden die Störungen in der Membranstruktur zwischen benachbarten Zellen durch einen elektrischen Durchbruchpuls ausgelöst (J. Membrane Biol. 67, 165–182 (1982), Electric Field-Induced Cell-to-Cell Fusion, U. Zimmermann and J. Vienken). Dabei werden – nach den bisherigen Modellvorstellungen – Löcher in der Membrankontaktzone benachbarter Zellen erzeugt, die zu einem zytoplasmatischen Kontinuum zwischen den beiden Zellen und zur Brückenbildung von Lipiden zwischen den Membranen der benachbarten Zellen führen. Die Lipidmoleküle ordnen sich nicht mehr in ihre ursprüngliche Membran ein. Sobald sich eine Brücke gebildet hat, kommt es aus energetischen Gründen zur Abrundung des entstandenen Gebildes, das aus den über die Lipidbrücken miteinander verbundenen Zellen besteht.

Zur Durchführung dieser bekannten Verfahren wird eine Kammer der eingangs bezeichneten Art eingesetzt. Die Elektroden der Kammer sind dabei zur Bildung der Zellenreihe an eine Einrichtung zur Erzeugung eines alternierenden elektrischen Feldes und zur Erzeugung des elektrischen Durchbruches an eine Einrichtung zur Erzeugung elektrischer Spannungspulse angeschlossen.

Bei der Durchführung der bekannten Verfahren ist man bestrebt, zur Erhöhung der Effektivität der Verfahren die in einer Suspension vorliegenden Zellen möglichst alle der elektrischen Behandlung zu unterwerfen. Dies setzt voraus, daß die in die Kammer eingegebenen Zellen möglichst alle dem elektrischen Feld ausgesetzt werden. Die toten Zonen in der Kammer sollten daher möglichst klein sein.

Es ist Aufgabe der Erfindung, eine Kammer der eingangs bezeichneten Art zu schaffen, bei der ein hoher Anteil der in die Kammer eingegebenen Zellen dem elektrischen Feld ausgesetzt wird. Die Kammer soll außerdem die gleichzeitige und gleichmäßige Behandlung einer großen Anzahl von Zellen ermöglichen.

Die der Erfindung zugrundeliegende Aufgabe wird gemäß der Erfindung dadurch gelöst, daß der Raum zur Aufnahme der die Zellen enthaltenden Suspension von einem Innenkörper und eine den Innenkörper um dessen Längsachse mit gleichbleibendem Abstand umschließenden Außenhülle seitlich begrenzt ist und daß die in den Raum hineinragenden Elektroden den Innenkörper in Form einer mehrgängigen Schraube mit gleichbleibender Steigung derart umgeben, daß der durch die Elektroden begrenzte Bereich als ein in Form einer mehrgängigen Schraube den Innenkörper um dessen Längsachse umgebender Teilbereich des Raumes ausgebildet ist.

Die Elektroden und der begrenzte Bereich sind beispielsweise in der Form einer Doppelhelix, einer Vierfach-Helix usw. ausgebildet.

Eine sehr vorteilhafte Ausführungsform der Kammer besteht darin, daß jede Elektrode nach beiden Seiten zur nächstliegenden anderen Elektrode den gleichen Abstand hat. Damit wird erreicht, daß der Feldverlauf zwischen zwei benachbarten Elektroden gleich ist, womit die effektive Länge der Elektroden praktisch verdoppelt ist.

Der längliche Innenkörper ist zweckmäßigerweise zylinderförmig und die Außenhülle als zylinderförmiger Mantel mit gemeinsamer Mittelachse ausgebildet. Der längliche Innenkörper kann jedoch auch abweichend von der idealen Kreisform beispielsweise einen ovalen oder auch quadratischen Querschnitt aufweisen, an den jeweils die innere Form der Außenhülle angepaßt ist.

Eine zweckmäßige Ausführungsform der Kammer gemäß der Erfindung besteht darin, daß die Elektroden an dem Innenkörper anliegen. Dabei ist es ferner zweckmäßig, die Elektrodenanschlüsse durch den Innenkörper nach außen zu führen.

Bei einer weiteren Ausführungsform der Kammer liegen die Elektroden an der Innenwand der Außenhülle an.

Bei allen Ausführungsformen der Kammer gemäß der Erfindung ist die innere Form der Außenhülle in einem möglichst geringen Abstand von 20 μm bis 500 μm – im allgemeinen jedoch nicht geringer als 100 μm – an die Form des Innenkörpers angepaßt, so daß das tote Volumen, also das Volumen, in welchem Zellen nicht dem elektrischen Feld ausgesetzt sind, möglichst klein ist. Dabei ist es zweckmäßig, daß der Abstand zwischen den Elektroden und der den Elektroden gegenüberliegenden Wandung – also beispielsweise bei am Innenkörper anliegenden Elektroden die Innenwandung der Außenhülle – so bemessen ist, daß die suspendierten Zellen und, im Falle einer Fusion von Zellen, die Fusionsprodukte den Durchgang zwischen Elektroden und Wandung gerade ohne Schädigung passieren können.

Bei Verwendung von Einrichtungen zur Erzeugung eines elektrischen Wechselfeldes (für die Bildung der Zellenreihe) mit einer Ausgangsspannung von 50 $V_{p-p}$ an 50 Ω und einer Einrichtung zur Erzeugung eines Rechteckpulses (für den elektrischen Durchbruch) von 100 V an 50 Ω beträgt der Abstand der Elektroden zueinander je nach der Art und Größe der zu behandelnden Zellen im allgemeinen 20 μm bis 500 μm. Er wird für Hefe und Bakterienzellen etwa zwischen 50 μm und 100 μm, für tierische Zellen etwa zwischen 100 μm und 200 μm und für pflanzliche Zellen etwa zwischen 100 μm und 500 μm liegen. Die Steigung der mehrgängigen Schraube ergibt sich dabei aus dem gewählten Elektrodenstand. Bei Verwendung einer Einrichtung mit höherer Ausgangsspannung und entsprechend höherer Leistung kann der Elektrodenabstand selbstverständlich größer – beispielsweise 5 mm – sein.

Bei größerem Elektrodenabstand und damit auch bei größerem Elektrodenquerschnitt ist von Vorteil, wenn die Elektroden kreisförmigen Querschnitt aufweisen. Das ist beispielsweise dann der Fall, wenn die Elektroden aus einem auf den Innenkörper gewickelten Draht bestehen. Infolge des Kreisquerschnittes bildet sich zwischen den Elektroden ein im Hinblick auf die Bildung einer Zellenreihe günstiges inhomogenes Feld aus. Dabei ist es zweckmäßig, daß die Elektroden zu etwa einem Drittel ihres Durchmessers in ihrer Auflage versenkt angeordnet sind. Auf diese Weise wird der Raum für die Zellensuspension möglichst klein gehalten.

Bei sehr geringen Elektrodenabständen, bei denen die Elektrodenschraube nicht aus Draht gewickelt werden kann, sondern andere Techniken zur Herstellung der Elektrodenschraube angewendet werden müssen, ergibt sich ein inhomogenes elektrisches Feld dann, wenn die Elektroden als flaches Band an dem Innenkörper oder der Innenwandung der Außenhülle anliegen.

Bei einer Elektrodenlänge von mehr als etwa 1 m sind die Elektroden zweckmäßigerweise jeweils als elektrische Schleife ausgebildet, wobei jedes Ende der Elektroden mit einem Elektrodenanschluß in Verbindung steht. Zur Vermeidung von Reflektionen im Elektrodensystem werden zwei entsprechende – einer Seite der mehrgängigen Schraube zugeordnete – Anschlüsse der Elektroden über einen Widerstand miteinander verbunden, wobei der Widerstand auf den Wellenwiderstand abgeglichen ist. Die übrigen Anschlüsse der Elektroden sind dann mit der oder den Einrichtungen zur Erzeugung des elektrischen Feldes verbunden.

Ein Abgleich des Widerstandes ist dabei beispielsweise – bei einer Außenhülle und einem Innenkörper aus Polymethacrylat und Platin als Elektrodenmaterial – bei einem Elektrodenabstand von 200 µm und einer Frequenz der elektrischen Spannung von 1 MHz sowie einer Leitfähigkeit der Suspensionslösung von etwa 0,5 bis 1 mS dann nicht erforderlich, wenn die Elektroden eine Länge von 1,5 m nicht überschreiten.

Um die Zellen während des Betriebes der Kammer unter dem Mikroskop beobachten zu können, ist es zweckmäßig, daß die Außenhülle der Kammer aus durchsichtigem Material, beispielsweise Polymethacrylat, besteht und an wenigstens einer Stelle ihres Außenmantels eben ausgebildet ist. Die Außenhülle kann dabei auch beispielsweise als Sechskant ausgebildet sein.

Während des Betriebes der Kammer kann es infolge des Stromflusses zu einer für die Zellen ungünstigen Erwärmung der die Zellen enthaltenden Suspension kommen. Dieser Erwärmung wird bei einer Ausführungsform der Kammer gemäß der Erfindung, bei der durch den Innenkörper eine Kühlleitung geführt ist, entgegengewirkt. Eine Kühlleitung kann aber auch in der Außenhülle angeordnet sein.

Die Kammer gemäß der Erfindung kann als Durchflußkammer ausgestaltet sein, beispielsweise dadurch, daß sich an den Stirnseiten der Kammer die Zu- und Ableitungen befinden. Zum Betrieb der Kammer wird dann eine vorbestimmte Menge an Zellsuspensionslösung in die Kammer gedrückt oder gesaugt, die elektrische Behandlung vorgenommen, darauf die in der Kammer befindliche Suspensionslösung aus der Kammer gedrückt oder gesaugt und durch eine neue Menge an Zellsuspension ersetzt.

Zum Durchspülen des Kammerraumes mit einer Reinigungslösung nach der elektrischen Behandlung der Zellen kann es zweckmäßig sein, daß der Innenkörper ein zum Raum der Kammer hin geöffnetes, von außen über eine Leitung zugängliches Porensystem aufweist, über welches eine Flüssigkeit in den Raum gedrückt werden kann, wobei die dem Raum zugewendeten Öffnungen der Poren kleiner als der Durchmesser der zu behandelnden Zellen ist. Während des Betriebs der Kammer ist das Porensystem dann zweckmäßigerweise mit der gleichen Lösung, in welcher die Zellen suspendiert sind, jedoch ohne Zellen, gefüllt.

Als besonders vorteilhaft hat sich jedoch eine Ausführungsform der Kammer gemäß der Erfindung erwiesen, bei der die Außenhülle der Kammer an einer Stirnseite geschlossen ausgeführt ist, der Innenkörper stabförmig und an die innere Form der Außenhülle angepaßt ausgebildet ist und daß ein Verschluß zum Verschließen des Raumes an der offenen Stirnseite der Außenhülle vorgesehen ist. Durchqueren dabei die Elektrodenanschlüsse nicht den Raum der Kammer, sind die Elektrodenanschlüsse also bei an dem Innenkörper anliegenden Elektroden durch den Innenkörper nach außen geführt, dann kann eine Beladung der Kammer mit Zellsuspension dadurch vorgenommen werden, daß in die Außenhülle, welche zweckmäßigerweise die Form eines Reagenzglases hat, eine solche Menge an Zellsuspension eingegeben wird, die bei in die Außenhülle eingetauchtem Innenkörper den Kammerraum gerade ausfüllt und daß durch behutsames Eintauchen des stabförmigen Innenkörpers die die Zellen enthaltende Flüssigkeit über den ganzen Raum der Kammer verteilt wird.

Zweckmäßig kann es ferner sein, daß die geschlossene Stirnseite der Außenhülle eine Öffnung aufweist. Ist diese verschließbar, dann ist die Möglichkeit gegeben, zunächst wie mit der die Form eines Reagenzglases aufweisenden Außenhülle (die keine untere Öffnung aufweist) zu verfahren, nach der Durchführung der elektrischen Behandlung jedoch die Zellensuspension durch die Öffnung abzusaugen.

Eine weitere vorteilhafte Ausführungsform der Kammer gemäß der Erfindung besteht darin, daß ein an die Außenhülle lösbar anschließbares, mit der Öffnung in der Außenhülle in Verbindung stehendes Zusatzgefäß vorgesehen ist.

Zweckmäßigerweise wird bei verschlossener Öffnung in der Außenhülle der Kammer zunächst die elektrische Behandlung der Zellen vorgenommen, sodann bei offener Öffnung das Zusatzgefäß an der Außenhülle befestigt und danach die Zellen durch Zentrifugieren – wobei die Kammer selbstverständlich von der oder den Einrichtungen zur Erzeugung des elektrischen Feldes abgetrennt ist – durch die Öffnung in das Zusatzgefäß gebracht. Das Zusatzgefäß kann dabei mit einer beliebigen, für die Weiterbehandlung der Zellen geeigneten Lösung, beispielsweise einem Nähr- oder einem Selektionsmedium, gefüllt sein.

Bei einer weiteren Ausführungsform der Kammer gemäß der Erfindung ist das Zusatzgefäß auch während der elektrischen Behandlung der Zellen an der Außenhülle der Kammer befestigt. Hierzu ist, wenn sich im Zusatzgefäß eine Lösung befindet, die während des Betriebs der Kammer nicht in den Raum der Kammer gelangen soll, die über die Öffnung in der Außenhülle führende Verbindung zwischen dem Raum der Kammer und dem Innenraum des Zusatzgefäßes zweckmäßigerweise unterbrechbar.

Das kann beispielsweise durch ein von außen betätigbares Absperrorgan für die Öffnung in der Stirnseite der Außenhülle geschehen.

Zur Unterbrechung der über die Öffnung in der Stirnseite der Außenhülle führenden Verbindung zwischen dem Raum der Kammer und dem Innenraum des Zusatzgefäßes kann die Öffnung des Zusatzgefäßes auch mit einer Folie abgedeckt sein. Dabei ist, um die Zellen nach deren elektrischen Behandlung durch Zentrifugieren in das Zusatzgefäß zu bringen, eine von außerhalb der Kammer betätigbare Einrichtung vorgesehen, mittels der die Folie bei an der Außenhülle befestigtem Zusatzgefäß durchlöchert werden kann.

Eine solche Einrichtung kann darin bestehen, daß eine bewegbar im Innenkörper geführte Nadel vorgesehen ist, mittels der die Folie durchlöchert werden kann. Eine solche Nadel kann jedoch auch im Zusatzgefäß, und zwar von unten betätigbar angebracht sein.

Das Volumen des Innenraums des Zusatzgefäßes wird zweckmäßigerweise so gewählt, daß für die nach der elektrischen Behandlung in das Zusatzgefäß abzentrifugierten Zellen die Zellendichte für das Wachstum hinreichend ist. Im allgemeinen wird das Volumen – das allerdings von der Größe der Kammer abhängig ist – 500 µl bis etwa 2 ml betragen.

Ausführungsbeispiele der Kammer gemäß der Erfindung sind in der Zeichnung schematisch dargestellt und werden im folgenden näher erläutert:
Es zeigen

Figur 1 einen Längsschnitt durch eine als Durchflußkammer ausgebildete Kammer mit am Innenkörper anliegenden Elektroden in Form einer Doppelhelix,

Figur 2 einen Längsschnitt durch eine als Durchflußkammer ausgebildete Kammer mit an der Innenwandung der Außenhülle anliegenden Elektroden in Form einer Doppelhelix,

Figur 3 Längsschnitt durch eine Kammer mit die Form eines Reagenzglases aufweisender Außenhülle und in die Außenhülle einschiebbarem Innenkörper,

Figur 4 Längsschnitt durch eine Kammer gemäß Figur 3 mit an die Außenhülle aufsteckbarem Zusatzgefäß und im Innenkörper bewegbar geführter Nadel zum Durchlöchern der Folie des Zusatzgefäßes,

Figur 5 Längsschnitt durch einen Innenkörper gemäß Figur 4 mit Kühlleitung,

Figur 6 Querschnitt durch den Innenkörper gemäß Figur 5 längs der Linie A–B.

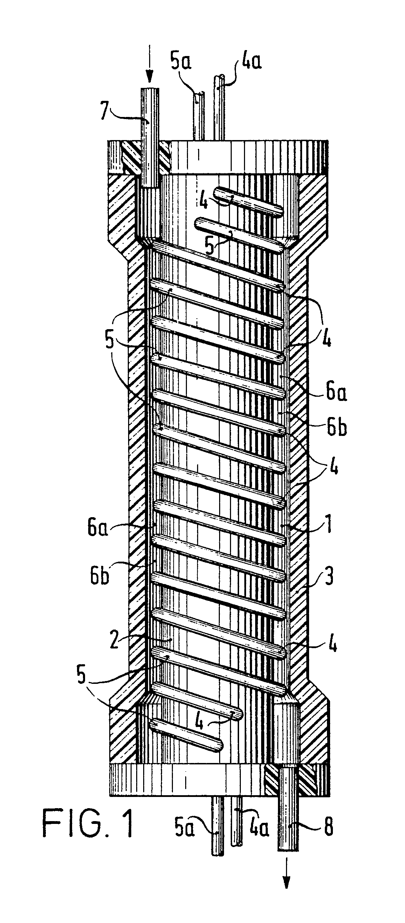

Wie aus Figur 1 ersichtlich ist, wird der Raum 1 der Kammer durch einen Innenkörper 2 und eine den Innenkörper um dessen Längsachse umschließende Außenhülle 3 seitlich begrenzt. Innenkörper 2 ist – aus der Zeichnung allerdings nicht erischtlich – zylinderförmig und die Außenhülle 3 als zylinderförmiger Mantel ausgebildet. Beide Körper bestehen aus Polymethacrylat oder einem anderen Kunststoff.

Wie aus Figur 1 ferner zu ersehen ist, umgeben die Elektroden 4 und 5, die aus einer Wicklung aus Platindraht bestehen, den Innenkörper 2. Die Elektroden haben auf ihrer ganzen Länge den gleichen Abstand zueinander. Sie sind jeweils als Schleife ausgebildet. Die vier Elektrodenanschlüsse 4a, 4b sowie 5a und 5b sind durch den Innenkörper 2 nach außen geführt. Die Anschlüsse 4b und 5b können über einen in der Zeichnung nicht dargestellten, abzugleichenden Widerstand miteinander verbunden werden.

Der durch die Elektroden 4 und 5 begrenzte Bereich, in dem die Zellen dem elektrischen Feld ausgesetzt werden, umgibt den Innenkörper 2 als ein in der Form einer Doppelhelix ausgebildeter Teilbereich 6a und 6b des Raumes 1.

Der Abstand der Elektroden 4 und 5 zu der Innenwandung der Außenhülle 3 ist über die ganze Länge der Kammer konstant und liegt in dem in der Zeichnung dargestellten Beispiel etwa in dem Bereich von 100 µm bis 500 µm.

Die in Figur 1 dargestellte, als Durchflußkammer ausgebildete Kammer ist an den Stirnflächen geschlossen. Zur Zuführung der die Zellen enthaltenden Lösung sind eine verschließbare Zuleitung 7 und eine verschließbare Ableitung 8 vorgesehen.

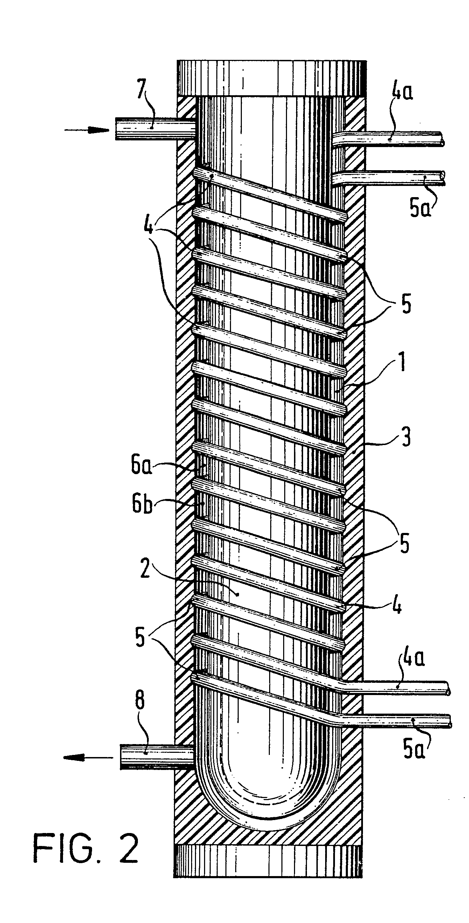

Bei der in Figur 2 dargestellten, ebenfalls als Durchflußkammer ausgebildeten Kammer liegen im Unterschied zu der in Figur 1 dargestellten Ausführungsform der Kammer die Elektroden 4 und 5 an der Innenwandung der Außenhülle 3 an.

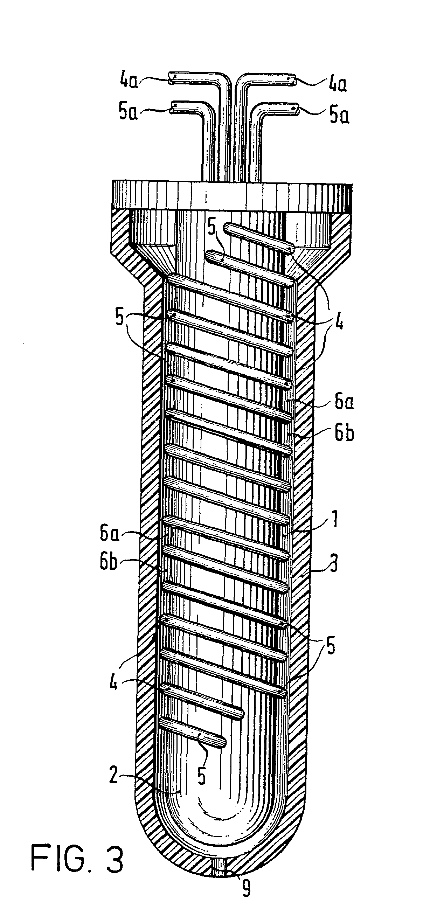

Bei der in Figur 3 dargestellten Ausführungsform der Kammer ist die Außenhülle 3 an einer Stirnseite bis auf eine verschließbare Öffnung 9 verschlossen ausgeführt. Die Außenhülle 3 ist zylinderförmig und – bis auf die Öffnung 9 – in der Art eines Reagenzglases ausgebildet. Der Innenkörper 2 ist stabförmig und an die Innenform der Außenhülle 3 angepaßt ausgebildet. An ihm liegen die Elektroden 4 und 5 an, deren Anschlüsse 4a, 4b sowie 5a und 5b durch den Innenkörper nach außen geführt sind. Der durch die Elektroden begrenzte Bereich umgibt, wie bei der in Figur 1 dargestellten Ausführungsform der Kammer, den Innenkörper 2 als in der Form einer Doppelhelix ausgebildeter Teilbereich 6a und 6b des Raumes 1.

An der der offenen Stirnseite der Außenhülle 3 entsprechenden Stirnseite des Innenkörpers 2 ist ein Verschluß 10 für die Außenhülle 3 angebracht.

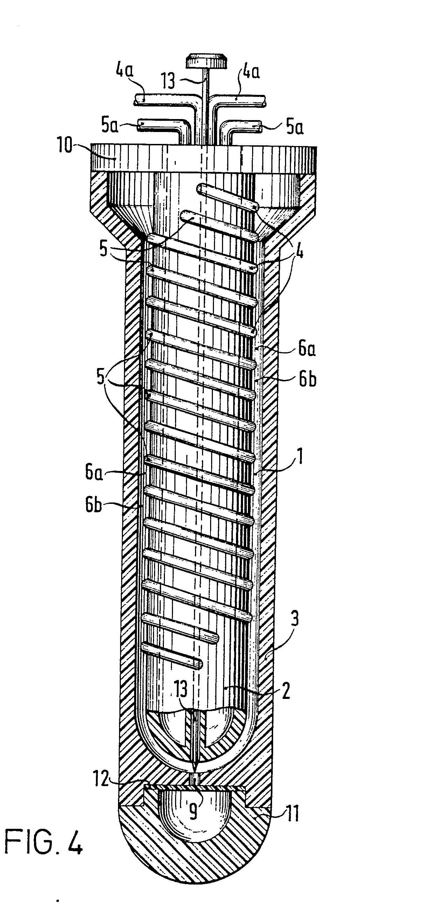

Die in Figur 4 dargestellte Ausführungsform der Kammer geht von der in Figur 3 dargestellten Ausführungsform aus, ist jedoch um das Zusatzgefäß 11 ergänzt. Die Außenhülle 3 ist bei der in Figur 4 dargestellten Ausführungsform so ausgebildet, daß das Zusatzgefäß 11 aufsteckbar ist. Die Öffnung 9 ist nicht verschlossen. Das Zusatzgefäß ist mit einer Folie 12 verschließbar. Es dient dann zur Aufnahme einer Flüssigkeit, beispielsweise eines Nährmediums, das während des Betriebs der Kammer nicht in den Raum 1 gelangen soll. Zum Durchlöchern der Folie nach dem Betrieb der Kammer und um die im Raum 1 befindlichen Zellen in das Zusatzgefäß abzentrifugieren zu können, ist eine durch den Innenkörper 2 geführte, bewegbare Nadel 13 vorgesehen.

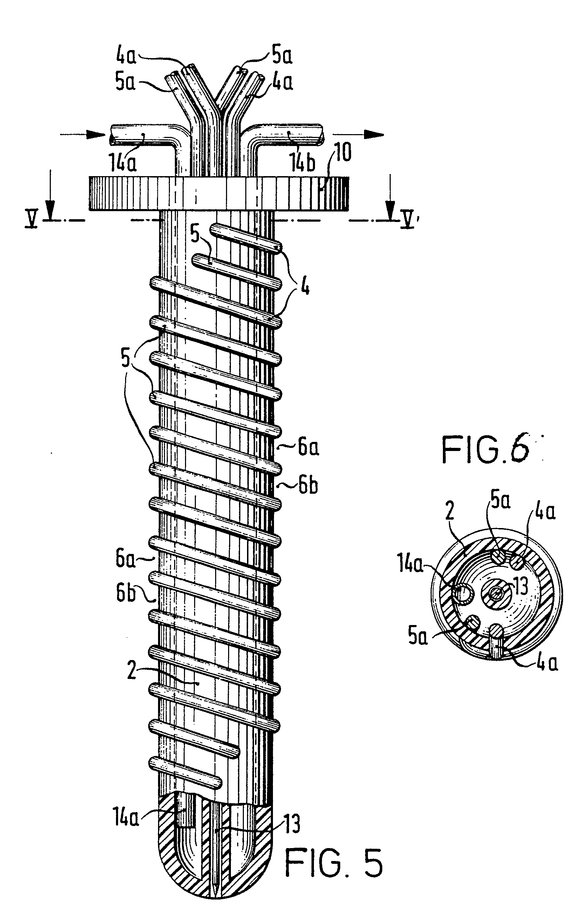

In Figur 5 ist der Innenkörper 2 gemäß der in Figur 4 dargestellten Ausführungsform der Kammer wiedergegeben, jedoch mit einem zusätzlichen Kühlsystem, durch die ein Kühlmedium während des Betriebs der Kammer geleitet werden kann. Das Kühlsystem besteht aus der Zuleitung 14a, dem Hohlraum des Innenkörpers, in den die Zuleitung 14a hineinragt, und der Ableitung 14b.

Figur 6 zeigt einen Querschnitt durch den Innenkörper 2 der Figur 5 längs der Linie A–B.

**Patentansprüche**

1. Kammer für die Behandlung von Zellen im elektrischen Feld, bei der ein aus elektrisch nicht leitenden Wänden gebildeter Raum zur Aufnahme der die Zellen enthaltenden Suspension vorgesehen ist, in den wenigstens zwei Elektroden derart hineinragen, daß ein zwischen den Elektroden liegender, durch diese begrenzter Bereich gebildet wird, in welchem die Zellen einem zwischen den Elektroden ausgebildeten elektrischen Feld ausgesetzt sind, dadurch gekennzeichnet, daß der Raum (1) von einem Innenkörper (2) und einer den Innenkörper um dessen Längsachse mit gleichbleibendem Abstand umschließenden Außenhülle (3) seitlich begrenzt ist und daß die in den Raum hineinragenden Elektroden (4 und 5) den Innenkörper in Form einer mehrgängigen Schraube mit gleichbleibender Steigung derart umgeben, daß der durch die Elektroden begrenzte Bereich als ein in Form einer mehrgängigen Schraube den Innenkörper um dessen Längsachse umgebender Teilbereich (6a, 6b) des Raumes (1) ausgebildet ist.

2. Kammer nach Anspruch 1, dadurch gekennzeichnet, daß jede Elektrode (4 und 5) nach beiden Seiten zur nächstliegenden anderen Elektrode den gleichen Abstand hat.

3. Kammer nach Anspruch 1 oder 2, dadurch gekennzeichnet, daß der Innenkörper (2) zylinderförmig und die Außenhülle (3) als zylinderförmiger Mantel mit gemeinsamer Mittelachse ausgebildet sind.

4. Kammer nach einem der vorhergehenden Ansprüche, dadurch gekennzeichnet, daß die Elektroden (4, 5) an dem Innenkörper (2) anliegen.

5. Kammer nach Anspruch 4, dadurch gekennzeichnet, daß die Elektrodenanschlüsse (4a, 4b, 5a, 5b) durch den Innenkörper (2) nach außen geführt sind.

6. Kammer nach einem der Ansprüche 1 bis 4, dadurch gekennzeichnet, daß die Elektroden (4, 5) an der Innenwand der Außenhülle (3) anliegen.

7. Kammer nach einem der vorhergehenden Ansprüche, dadurch gekennzeichnet, daß der Abstand zwischen den Elektroden (4, 5) und der den Elektroden gegenüberliegenden Wandung so bemessen ist, daß die suspendierten Zellen und, im Falle einer Fusion von Zellen, die Fusionsprodukte den Durchgang zwischen Elektroden (4, 5) und der Wandung gerade ohne Schädigung passieren können.

8. Kammer nach einem der vorhergehenden Ansprüche, dadurch gekennzeichnet, daß der Abstand zwischen den Elektroden (4 und 5) je nach der Art und Größe der zu behandelnden Zellen 20 µm bis 500 µm beträgt.

9. Kammer nach einem der vorhergehenden Ansprüche, dadurch gekennzeichnet, daß die Elektroden (4, 5) bei größerem Elektrodenabstand einen kreisförmigen Querschnitt aufweisen.

10. Kammer nach Anspruch 9, dadurch gekennzeichnet, daß die Elektroden (4, 5) zu etwa einem Drittel ihres Durchmessers in ihrer Auflage versenkt angeordnet sind.

11. Kammer nach einem der vorhergehenden Ansprüche, dadurch gekennzeichnet, daß bei einer Elektrodenlänge von mehr als 1 m die Elektroden (4, 5) jeweils als elektrische Schleife ausgebildet und jedes Ende der Elektroden (4, 5) mit einem Elektrodenanschluß (4a, 4b, 5a, 5b) in Verbindung steht.

12. Kammer nach einem der vorhergehenden Ansprüche, dadurch gekennzeichnet, daß die Außenhülle (3) aus durchsichtigem Material besteht und an wenigstens einer Stelle ihres Außenmantels eben ausgebildet ist.

13. Kammer nach einem der vorhergehenden Ansprüche, dadurch gekennzeichnet, daß durch den Innenkörper (2) eine Kühlleitung (14) geführt ist.

14. Kammer nach einem der vorhergehenden Ansprüche, dadurch gekennzeichnet, daß der Innenkörper (2) ein zum Raum (1) der Kammer hin geöffnetes, von außen über eine Leitung zugängliches Porensystem aufweist, über welches eine Flüssigkeit in den Raum (1) gedrückt werden kann, wobei die dem Raum zugewendeten Öffnungen der Poren kleiner sind als der Durchmesser der zu behandelnden Zellen.

15. Kammer nach einem der vorhergehenden Ansprüche, dadurch gekennzeichnet, daß die Außenhülle (3) an einer Stirnseite geschlossen ausgeführt ist, der Innenkörper (2) stabförmig und an die innere Form der Außenhülle (3) angepaßt ausgebildet ist und daß ein Verschluß (10) zum Verschließen des Raumes (1) an der offenen Stirnseite der Außenhülle (3) vorgesehen ist.

16. Kammer nach Anspruch 15, dadurch gekennzeichnet, daß die geschlossene Stirnseite der Außenhülle (3) eine Öffnung (9) aufweist.

17. Kammer nach Anspruch 16, dadurch gekennzeichnet, daß die Öffnung (9) verschließbar ist.

18. Kammer nach einem der Ansprüche 16 und 17, dadurch gekennzeichnet, daß ein an die Außenhülle (3) lösbar anschließbares, mit der Öffnung (9) in der Außenhülle in Verbindung stehendes Zusatzgefäß (11) vorgesehen ist.

19. Kammer nach Anspruch 18, dadurch gekennzeichnet, daß die über die Öffnung (9) in der Stirnseite der Außenhülle (3) führende Verbindung zwischen dem Raum (1) der Kammer und dem Innenraum des Zusatzgefäßes (11) unterbrechbar ist.

20. Kammer nach Anspruch 19, dadurch gekennzeichnet, daß zur Unterbrechung der über die Öffnung (9) führenden Verbindung zwischen dem Raum (1) der Kammer und dem Innenraum des Zusatzgefäßes (11) die Öffnung des Zusatzgefäßes mit einer Folie (12) abgedeckt ist.

21. Kammer nach Anspruch 20, dadurch gekennzeichnet, daß eine von außerhalb der Kammer betätigbare Einrichtung (13) vorgesehen ist, mittels der die Folie (12) bei an der Außenhülle (3) befestigtem Zusatzgefäß (11) durchlöchert werden kann.

## Claims

1. Chamber for the treatment of cells in an electric field, in which a space formed by electrically non-conductive walls is provided to hold the suspension containing the cells into which project at least two electrodes in such a way that a region is formed, which lies between the electrodes and is bounded by them, in which the cells are exposed to an electric field formed between the electrodes, characterized in that the space (1) is bounded at its sides by an inner body (2) and an outer casing (3) enclosing the inner body around its longitudinal axis at a constant distance and that the electrodes (4 and 5) projecting into the space surround the inner body in the form of a multiple screw thread of constant pitch in such a way that the region bounded by electrodes is formed as a subregion (6a, 6b) of the space (1) in the form of a multiple screw thread surrounding the inner body around its longitudinal axis.

2. Chamber according to Claim 1, characterized in that each electrode (4 and 5) is at the same distance from the other adjacent electrode on either side.

3. Chamber according to Claim 1 or 2, characterized in that the inner body (2) is constructed cylindrically and the outer casing (3) is constructed as a cylindrical shell with a common central axis.

4. Chamber according to one of the preceding claims, characterized in that the electrodes (4, 5) are seated against the inner body (2).

5. Chamber according to Claim 4, characterized in that the electrode connections (4a, 4b, 5a, 5b) are taken to the outside through the inner body (2).

6. Chamber according to one of Claims 1 to 4, characterized in that the electrodes (4, 5) are seated against the inside wall of the outer casing (3).

7. Chamber according to one of the preceding claims, characterized in that the distance between the electrodes (4, 5) and the side opposite the electrodes is dimensioned so that the suspended cells and, in the case of fusion of cells, the fusion products can pass straight through the passage between the electrodes (4, 5) and the side without damage.

8. Chamber according to one of the preceding claims, characterized in that the distance between the electrodes (4 and 5) is 20 µm to 500 µm depending on the type and size of the cells to be treated.

9. Chamber according to one of the preceding claims, characterized in that, with larger distances between the electrodes, the electrodes (4, 5) have circular cross-sections.

10. Chamber according to Claim 9, characterized in that the electrodes (4, 5) are positioned so that they are embedded up to approximately one third of their diameter into their support.

11. Chamber according to one of the preceding claims, characterized in that for an electrode length of more than 1 m the electrodes (4, 5) are each formed as an electric loop and each of the ends of the electrodes (4, 5) are connected to an electrode connection (4a, 4b, 5a, 5b).

12. Chamber according to one of the preceding claims, characterized in that the outer casing (3) consists of transparent material and is made flat on at least one place on its outer shell.

13. Chamber according to one of the preceding claims, characterized in that a cooling line (14) is passed through the inner body (2).

14. Chamber according to one of the preceding claims, characterized in that the inner body (2) has a pore system, which opens into the chamber space (1) and is accessible from outside by a line, through which a liquid can be forced into the space (1), the openings of pores facing the space being smaller than the diameter of the cells to be treated.

15. Chamber according to one of the preceding claims, characterized in that the outer casing (3) is designed with one end closed, the inner body (2) is rodshaped and adapted to the internal form of the outer casing (3) and that a closure (10) for closing off the space (1) is provided at the open end of the outer casing (3).

16. Chamber according to Claim 15, characterized in that the closed end of the outer casing (3) has an opening (9).

17. Chamber according to Claim 16, characterized in that the opening (9) can be closed.

18. Chamber according to one of Claims 16 and 17, characterized in that an auxiliary vessel (11) is provided which can be detachably joined to the outer casing (3) and is connected to the opening (9) in the outer casing.

19. Chamber according to Claim 18, characterized in that the connection between the chamber space (1) and the internal space of the auxiliary vessel (11) leading via the opening (9) in the end of the outer casing (3) can be shut off.

20. Chamber according to Claim 19, characterized in that to shut off the connection between the chamber space (1) and the internal space of the auxiliary vessel (11) leading via the opening (9) the opening in the auxiliary vessel is covered with a film (12).

21. Chamber according to Claim 20, characterized in that a device (13) which can be actuated from outside the chamber is provided by which the film (12) for the auxiliary vessel (11) attached to the outer casing (3) can be punctured.

## Revendications

1. Enceinte de traitement de cellules dans un champ électrique, dans laquelle il est prévu une chambre, à parois non conductrices de l'électricité, de réception de la suspension contenant les cellules, dans laquelle pénétrent au moins deux électrodes de manière à former une région délimitée par les électrodes, comprise entre celles-ci et dans laquelle les cellules sont soumises à un champ électrique formé entre les électrodes, caractérisée en ce que la chambre (1) est délimitée latéralement par une pièce intérieure (2) et par une douille extérieure (3) entourant la pièce intérieure à distance constante de son axe longitudinal et en ce que les électrodes (4 et 5) pénétrant dans la chambre entourent la pièce intérieure sous la forme d'un vis à filets multiples de pas constant de manière à constituer la région délimitée par les électrodes d'une région partielle (6a, 6b) de la chambre (1), ayant la forme d'une vis à filets multiples et entourant l'axe longitudinal de la pièce intérieure.

2. Enceinte suivant la revendication 1, caractérisée en ce que chaque électrode (4 et 5) est à la même distance des deux côtés de l'autre électrode la plus proche.

3. Enceinte suivant la revendication 1 ou 2, caractérisée en ce que la pièce intérieure (2) est de forme cylindrique et le douille extérieure (3) est constituée sous la forme d'une enveloppe cylindrique et leur axe médian est commun.

4. Enceinte suivant l'une des revendications précédentes, caractérisée en ce que les électrodes (4, 5) sont appliquées à la pièce intérieure (2).

5. Enceinte suivant la revendication 4, caractérisée en ce que les bornes d'électrodes (4a, 4b, 5a, 5b) sont guidées vers l'extérieur en passant à travers la pièce intérieure (2).

6. Enceinte suivant l'une des revendications 1 à 4, caractérisée en ce que les électrodes (4, 5) sont appliquées à la paroi intérieure de la douille extérieure (3).

7. Enceinte suivant l'une des revendications précédentes, caractérisée en ce que la distance entre les électrodes (4, 5) et la paroi opposée aux électrodes est telle que les cellules en suspension et, dans le cas d'une fusion de cellules, les produits de la fusion peuvent passer directement sans dommage dans le passage compris entre les électrodes (4, 5) et la paroi.

8. Enceinte suivant l'une des revendications précédentes, caractérisée en ce que la distance entre les électrodes (4 et 5) est comprise entre 20 microns et 500 microns, suivant la nature et la dimension des cellules à traiter.

9. Enceinte suivant l'une des revendications précédentes, caractérisée en ce que les électrodes (4, 5) ont, dans le cas d'une grande distance entre les électrodes, une section transversale circulaire.

10. Enceinte suivant la revendication 9, caractérisée en ce que les électrodes (4, 5) sont encastrées dans leur support sur environ un tiers de leur diamètre.

11. Enceinte suivant l'une des revendications précédentes, caractérisée en ce que, pour une longueur d'électrode supérieure à 1 m, les électrodes (4, 5) sont constituées sous la forme d'une boucle électrique et chaque extrémité des électrodes (4, 5) communique avec une borne d'électrodes (4a, 4b, 5a, 5b).

12. Enceinte suivant l'une des revendications précédentes, caractérisée en ce que la douille extérieure (3) est en matériau transparent et est plane, au moins en un endroit de sa surface latérale extérieure.

13. Enceinte suivant l'une des revendications précédentes, caractérisée en ce qu'un conduit de refroidissement (14) passe dans la pièce intérieure (2).

14. Enceinte suivant l'une des revendications précédentes, caractérisée en ce que la pièce intérieure (2) comporte un système de pores, qui débouche sur la chambre (1) de l'enceinte, qui est accessible de l'extérieur par un conduit et par lequel un liquide peut être refoulé dans la chambre (1), les ouvertures des pores tournées vers la chambre étant inférieures au diamètre des cellules à traiter.

15. Enceinte suivant l'une des revendications précédentes, caractérisée en ce qu'un côté frontal de la douille extérieure (3) est fermé, la pièce intérieure (2) est en forme de bâtonnet et est adaptée à la forme intérieure de la douille extérieure (3) et il est prévu une fermeture (10) pour fermer la chambre (1) du côté frontal ouvert de la douille extérieure (3).

16. Enceinte suivant la revendication 15, caractérisée en ce que le côté frontal fermé de la douille extérieure (3) comporte une ouverture (9).

17. Enceinte suivant la revendication 16, caractérisée en ce que l'ouverture (9) peut être fermée.

18. Enceinte suivant l'une des revendications 16 ou 17, caractérisée en ce qu'il est prévu un récipient supplémentaire (11) qui peut être raccordé de manière amovible à la douille extérieure (3), et qui communique avec l'ouverture (9) de la douille extérieure.

19. Enceinte suivant la revendication 18, caractérisée en ce que la communication entre la chambre (1) de l'enceinte et l'intérieur du récipient supplémentaire (11), en passant par l'ouverture (9) ménagée sur le côté frontal de la douille extérieure (3), peut être interrompue.

20. Enceinte suivant la revendication 19, caractérisée en ce que, pour interrompre la communication entre la chambre (1) de l'enceinte et l'intérieur du récipient supplémentaire (11) en passant par l'ouverture (9), l'ouverture du récipient supplémentaire est recouverte d'une feuille (12).

21. Enceinte suivant la revendication 20, caractérisée en ce qu'il est prévu un dispositif (13) qui peut être manœuvré de l'extérieur de l'enceinte et au moyen duquel la feuille (12) du récipient supplémentaire (11) fixé à la douille extérieure (3) peut être perforée.

FIG. 1

FIG. 2

4a
5a
4a
5a

5

5

5

4

6a
6b

1

3

6a
6b

5

4

2

5

9

FIG. 3

FIG. 4

FIG.6

FIG. 5